# EUROPEAN PATENT APPLICATION

(11) **EP 3 485 724 A1**
(43) Date of publication of application: **22.05.2019**
(21) Application number: 16906170.2
(22) Date of filing: 21.12.2016
(51) Int. Cl.: A01H 1/02, A01H 1/04, A01H 1/08, A01H 4/00

(54) **METHOD FOR BREEDINGBRASSICA NAPUS**

(30) Priority: 23.06.2016 CN 201610458280
(71) Applicant: Chengdu Academy of Agriculture and Forestry Sciences, Chengdu, Sichuan 611130 (CN)
(72) Inventor: FU, Shaohong, Chengdu Sichuan 611130 (CN); LI, Yun, Chengdu Sichuan 611130 (CN); YANG, Jin, Chengdu Sichuan 611130 (CN); WANG, Jisheng, Chengdu Sichuan 611130 (CN); ZOU, Qiong, Chengdu Sichuan 611130 (CN); TAO, Lanrong, Chengdu Sichuan 611130 (CN); KANG, Zeming, Chengdu Sichuan 611130 (CN); TANG, Rong, Chengdu Sichuan 611130 (CN)
(74) Representative: Roman, Alexis
(86) International application number: PCT/CN2016/111327
(87) International publication number: WO 2017/219633

(57) **Abstract**

The present invention discloses a method for breeding *Brassica napus* varieties and materials with a double haploid induction line of rapeseed, including: 1) determining target traits of breeding restorer lines, maintainer lines and conventional varieties of *Brassica napus;* 2) crossing or convergently crossing two or more *Brassica napus* with the target traits; 3) pollinating the cross or back-cross progenies with the double haploid induction line of rapeseed; 4) identifying the stability of the induced progenies; 5) performing test-cross identification or yield and resistance identification on the stable progenies; and 6) forming stable restorer lines and maintainer lines for cross breeding combination or for forming conventional varieties. The present invention can quickly and efficiently obtain rapeseed materials or conventional varieties with application value in breeding on a large scale, and lays a solid foundation for genetic breeding of *Brassica napus,* innovation of breeding resources, breeding of conventional rapeseed varieties, and breeding of new varieties of hybrid rapeseed. The method of the present invention can greatly improve the breeding speed and efficiency of hybrid or conventional varieties of *Brassica napus,* and reduce the human and material resources.

## Description

### BACKGROUND

### Technical Field

The present invention relates to agriculture, and in particular to a method for breeding *Brassica napus* varieties and materials with a double haploid induction line of rapeseed.

### Related Art

Rapeseed is a major oil crop in China. *Brassica napus* is a complex species evolved from *Brassica campestris* (aa, n=10) and *Brassica oleracea* (cc, n=9) by natural inter-species crossing and then doubling diploidization. The *Brassica napus* is judged from a chromosome source to be tetraploid (2n=38), and is the main type of cultivated rapeseed in present rapeseed production. For breeding a new variety of *Brassica napus,* a new inbred line or genetically stable homozygous strains-homozygous line (inbred line) is bred first, e.g., these homozygous strains meet the production requirements in resistance, yield, quality, etc., and a new rapeseed variety (conventional variety) is finally identified or approved by regional trials. Second, the homozygous strains are test-crossed with a sterile line to judge a restoring and maintaining relationship. If a restorer line is crossed with a sterile line to test-match a new hybrid variety, if a maintainer line is test-matched with a sterile line to breed a new sterile line with the characteristics of the maintainer line, if stable strains are neither restored nor maintained (the test-match progenies cannot completely restore the fertility or cannot completely maintain highly sterile) and cannot form conventional varieties used in production, such homozygous strains are either eliminated, or crossed with other maintainer line or restorer line to enter next round of breeding of breeding materials. Under normal circumstances, the breeding of a conventional rapeseed variety through conventional artificial crossing needs 6-7 generations. If hybrid varieties are bred, stable sterile lines, maintainer lines and restorer lines need to be bred. The breeding time of new rapeseed varieties is longer, and is 10-15 years. The breeding of a conventional new rapeseed line is realized in such a manner that two or more lines with different genetic backgrounds are crossed, convergently crossed or back-crossed to form a hybrid F₁ generation (or a back-cross generation, and multi-generation back crossing can be performed according to the selection requirements of target traits to form BC2, BC3, ...), the back-cross progenies or F₁ generation is selfed to form an F₂ generation, excellent individual plants are selected from the F₂ generation and selfed to form an F₃ generation, individual plants are selected from F₃ and selfed, and a new stable rapeseed line can be obtained till F₆ to F₇, which takes about 7-8 years, calculated by one generation per year, and also needs about 4 years through remote generation adding.

At present, inducing lines or double haploid inducing lines have not been reported in rapeseed. The "inducing lines" indicate that the pollen of a kind of plants as male parents is used to pollinate the same kind of plants, and the same kind of plants (female parents) can be induced to produce the corresponding effects, e.g., produce haploids, double haploids (DH lines), etc. Maize is mostly used among plants for breeding new varieties by inducing lines, but the inducing lines in maize are only haploid inducing lines. The earliest maize haploid inducing line was stock6, which can induce maize to produce only haploids, and then the haploid plants were doubled by artificial chromosomes to form homozygous diploids (double haploids), and the inducing efficiency is low, generally 10% or less (calculated by the number of haploids obtained from harvested seeds).

### SUMMARY

The object of the present invention is to provide a method for rapidly breeding restorer lines and maintainer lines of genetically stable *Brassica napus* or breeding *Brassica napus* varieties and materials with conventional varieties of rapeseed diploid inducing lines.

The object of the present invention is achieved in this way:
A method for breeding *Brassica napus* varieties and materials with a double haploid induction line of rapeseed according to the present invention, comprising the following steps:
1) determining target traits of breeding restorer lines, maintainer lines and conventional varieties of *Brassica napus,* crossing or convergently crossing at least two *Brassica napus* with the target traits, and performing back crossing or multi-generation back crossing according to the requirements of the target traits to form cross progenies, convergent cross progenies or back-cross progenies;
2) artificially castrating buds of the cross, convergent cross or back-cross progeny materials obtained in step 1) at the flowering stage, and performing bagging isolation;
3) artificially pollinating the plants within 2 to 4 days after castration in step 2) with pollen of the double haploid induction line of rapeseed, performing bagging isolation, and harvesting pollinated induced seeds;
4) planting individual plant induced seeds obtained in step 3), identifying the ploidies with a flow cytometer at the seedling stage to eliminate polyploids, haploids or plants with dominant characters of the double haploid induction line of rapeseed, selecting tetraploid plants with normal fertility, and bagging and selfing individual plants;
5) performing strain planting on selfing seeds of the tetraploid individual plants with normal fertility obtained in step 4), investigating the morphologic consistency of the strains, and identifying the consistency and stability of the strains through molecular markers (SSR or SRAP);
6) test-crossing the stable tetraploid strains identified in step 5) with *Brassica napus* cytoplasmic (polima cytoplasmic male sterile (CMS), ogura CMS, Hau CMS, JA CMS) sterile line, or with a *Brassica napus* genetic male sterile (GMS) line, identifying the fertility of the test-cross progenies, and judging the restoring and maintaining relationship of the test-cross male parents;
7) determining that the corresponding test-cross male parents are of a maintainer line if the test-cross progenies in step 6) are completely sterile, and are of a restorer line if the test-cross progenies are completely fertile;
8) continuing to back-cross the maintainer line identified by test-cross in step 7) with a sterile line by multiple generations to breed a stable sterile line consistent with the maintainer line in nuclear genes; directly test-matching the restorer line identified by test-cross with a sterile line of a corresponding system to breed a hybrid combination, and performing variety comparison test on the hybrid combination, wherein the variety that has yield, resistance, productivity and quality traits better than other large-area varieties in production and meets the variety identification (or approval) standards can form a hybrid rapeseed variety, which can be promoted and applied in production by identification (or approval) of provincial or national seed management departments; and
9) performing comparison and production trials on the stable strains obtained in step 6), wherein the variety that has yield, resistance, productivity and quality traits superior to the control and meets the variety identification (or approval) standards can form a conventional variety, which can be promoted and applied in production by identification (or approval) of provincial or national seed management departments.

A method for breeding the above-mentioned double haploid induction line of rapeseed comprises the following steps:
(1) breeding an early generation stable line with the parthenogenesis genetic characteristic:
   a. artificially doubling chromosomes of hybrid F₁ generation seeds of two rapeseed parent materials on a medium by using a chromosome doubling inducer to obtain doubled F₁ generation plants;
   b. selfing or forcedly selfing the doubled F₁ generation plants to obtain an F₂ generation, performing field planting observation on the F₂ generation, identifying the fertility of each individual plant, selecting fertile progenies and selfing same to obtain an F₃ generation, identifying the homozygosity of the F₃ generation by morphology, cytology and molecular markers, performing polymerase chain reaction amplification on progenies DNA, and observing the type and number of DNA bands of the individual plants under the amplification of each specific primer by electrophoresis, which shows that each individual plant is a hybrid progeny of two parents, and the molecular marker maps of the individual plants are consistent, indicating that these individual plants are of a homozygous line, i.e. an early generation stable line;
   c. reciprocally crossing the obtained early generation stable line with at least 10 conventional homozygous stable lines of rapeseed, and identifying the genetic characteristics of the early generation stable line at the F₁ and F₂ generations, i.e., identifying whether there is the parthenogenesis characteristic, wherein if F₁ is separated and part of stable strains appear in the F₂ generation in the reciprocal crossing, the corresponding early generation stable line is an early generation stable line with the parthenogenesis genetic characteristic;
(2) breeding polyploid rapeseed with dominant genetic traits, parthenogenesis genetic characteristic and ploidy genetic stability:
   a. crossing the early generation stable line with the parthenogenesis genetic characteristic with rapeseed with dominant traits (e.g. dominant dwarf, purple leaf, mottled leaf, yellow leaf, high erucic acid, etc.) to obtain hybrid F₁ generation seeds, and artificially doubling chromosomes of the hybrid F1 seeds on a medium by using a chromosome doubling inducer to obtain doubled F₁ plants with dominant traits;
   b. identifying the chromosome ploidies of the doubled F₁ plants with dominant traits through microscopic observation or a flow cytometer, selecting polyploid plants with dominant traits, and eliminating abnormal doubled plants, aneuploid plants and doubled plants without dominant traits, the polyploid plants with dominant traits being mainly hexaploid or octoploid rapeseed plants with ploidy genetic stability, good setting property, parthenogenesis genetic characteristic and dominant traits (e.g. dominant dwarf, purple leaf, mottled leaf, yellow leaf, high erucic acid, etc.);
(3) identifying the double haploid induction line of rapeseed and measuring the inducing capability:
   a. the dominant traits in the polyploid plants with ploidy genetic stability, parthenogenesis genetic characteristic and dominant traits can be used for removing hybrid plants generated in the test-cross progenies, and if dominant plants or aneuploid plants appear in the test-cross progenies, it indicates that the plants are generated by the polyploid plants and female parents and are removed; and
   b. if the individual plant test-cross progenies are completely sterile but have normal ploidies, i.e. diploid or tetraploid rapeseed, and do not have dominant traits, it indicates that the genes of the corresponding male parents of the test-cross progenies do not enter the test-cross progenies, wherein the dominant polyploid plants are of the double haploid induction line of rapeseed. The double haploid induction line of rapeseed can directly induce rapeseed to produce double haploid progenies without artificial chromosome doubling for obtaining homozygous lines, and has high inducing efficiency, which is up to 100%, generally 50% or more. The possible principle that the double haploid inducing line induces female plants to produce double haploids is that the inducing line can induce chromosome doubling and parthenogenesis in megaspore germ cells (egg cells) of female plants, i.e., double haploids are generated by parthenogenesis after the egg cells are doubled, and the extract mechanism of such a phenomenon is still unclear.

Stable genetic progenies of rapeseed are obtained by the method according to the present invention, wherein the double haploid induction line of rapeseed can induce parthenogenesis of female plants at the F₁ generation, stable double haploid individual plants are formed at the F₂ generation, the stability and the consistency are identified at the F₃ generation, and the stable genetic progenies are thus obtained. The method can be used for rapidly and effectively obtaining stable homozygous rapeseed lines by only three generations (2 years or 3 years), thereby improving the efficiency and pertinence of breeding *Brassica napus* materials and conventional rapeseed varieties. *Brassica napus* is the most widely used rapeseed cultivated species in production at present. 90% or more of promoted *Brassica napus* are hybrid varieties. The breeding of hybrid varieties is mainly based on the breeding of sterile lines (corresponding to maintainer lines) and restorer lines. The crossing of sterile lines and restorer lines realizes the utilization of heterosis, and forms hybrid varieties with excellent yield improvement potential, disease resistance and lodging resistance in production; the key to breed hybrid varieties of *Brassica napus* is to breed and aggregate multiple restorer lines and maintainer lines with excellent traits and genetic stability, which needs a long time period and consumes a lot of manpower and material resources. Therefore, it is difficult to breed excellent hybrid varieties of *Brassica napus.*

The above double haploid induction line of rapeseed is obtained by artificially doubling chromosomes of hybrid F₁ generation seeds of two parent materials, or hybrid F₁ generation seeds obtained by crossing the early generation stable line with the parthenogenesis genetic characteristic with rapeseed with dominant traits, on a medium by using a chromosome doubling inducer, and the specific method is as follows:
1) disinfecting the surfaces of the seeds with 75% alcohol for 25-40 seconds, disinfecting same with 0.1% mercury bichloride for 12-17 minutes, then washing away the mercury bichloride on the surfaces of the seeds with sterile water, sucking the water on the surfaces of the seeds with sterile paper, and then inoculating a first medium with the seeds;
2) allowing the seeds to root and sprout on the first medium under the culture conditions: temperature 23-25°C, daylight illumination 12-16 hours, light intensity 2000-3000 lux, night dark culture 8-12 hours, until the plants grow to 1-2 true leaves, and cutting the plants from the hypocotyls for continuing to grow on a second medium;
3) inserting the cut plants into the second medium to continue the culture, and after lateral buds are differentiated, transferring the lateral buds and the plants to a third medium for rooting culture; and
4) hardening seedlings of the plants at room temperature for 3-7 days after the plants grow thick roots after two weeks of rooting culture, taking the plants out, washing away the medium on the plants with tap water, soaking the plants in a soaking buffer solution for 15-30 minutes, and then transplanting the plants to a greenhouse, the greenhouse having a temperature of 16-25°C and a relative humidity of 60-80%, which can ensure that the survival rate of transplanting is 95% or above;
the first medium consists of the following components:

| | |
|---|---|
| MS medium | 1 L |
| 6-benzyl adenine | 0.5-1.5 mg |
| chromosome doubling inducer | 30-70 mg |
| sucrose | 20-30 g |
| agar | 8-10 g, |

the pH value of the first medium is 5.8-6.0;
the second medium consists of the following components:

| | |
|---|---|
| MS medium | 1 L |
| 6-benzyl adenine | 0.5-1 mg |
| chromosome doubling inducer | 20-40 mg |
| sucrose | 20-30 g |
| agar | 8-10 g, |

the pH value of the second medium is 5.8-6.0;
the third medium consists of the following components:

| | |
|---|---|
| MS medium | 1 L |
| α-naphthaleneacetic acid | 0.03-0.5 mg |
| chromosome doubling inducer | 5-20 mg |
| sucrose | 20-30 g |
| agar | 8-10 g, |

the pH value of the third medium is 5.8-6.0;
the soaking buffer solution consists of the following components:

| | |
|---|---|
| water | 1 L |
| famoxadone or curzate | 0.6-1.2 g |
| α-naphthaleneacetic acid | 0.5-1 mg. |

The above chromosome doubling inducer is at least one of colchicine, trifluralin and oryzalin.

The method described above can be rapidly applied to the breeding of hybrid varieties of *Brassica napus,* especially rapid breeding of restorer line and maintainer line materials, and can also be applied to rapid breeding of conventional varieties. The above materials or varieties can be obtained within 2 years or 3 generations, so that the breeding time of rapeseed is greatly saved and the breeding efficiency is improved.

The method of the present invention can rapidly breed new materials or varieties of hybrid *Brassica napus,* particularly has great application potential in the breeding of restorer lines and maintainer lines of *Brassica napus,* can obtain genetically stable *Brassica napus* CMS (polima CMS, ogura CMS, Hau CMS, JA CMS) restorer lines and maintainer lines fastest within 3 generations (2 years), can form new combinations (new varieties) of hybrid rapeseed within 4 generations (2-4 years), and can also obtain *Brassica napus* GMS restorer lines fastest within 3 generations. The present invention can also rapidly breed conventional varieties of *Brassica napus* with production potential through 3 generations.

The method of the present invention has the following advantages:
1. The method can rapidly (2 years or 3 generations) breed parent materials (restorer lines, maintainer lines) of *Brassica napus* hybrid varieties, and breed new combinations of hybrid rapeseed with promote potential within 4 generations (2-4 years), thereby greatly improving the breeding speed and efficiency of *Brassica napus* hybrid varieties;
2. The method can rapidly (2 years or 3 generations) breed conventional varieties of *Brassica napus* on a large scale, thereby greatly improving the breeding speed and efficiency of *Brassica napus* varieties;
3. The method can be applied to the utilization ways of different heterosis in breeding of *Brassica napus,* especially hybrid varieties, and can be applied to *Brassica napus* cytoplasmic male sterile lines (polima CMS, ogura CMS, Hau CMS, JA CMS), and *Brassica napus* genetic male sterile (GMS) lines;
4. The double haploid induction line of rapeseed directly induces female plants to produce double haploids without artificial chromosome doubling, and the double haploids can further form stable progenies in one step.

### BRIEF DESCRIPTION OF THE DRAWINGS

FIG. 1 is a flowchart of breeding of restorer lines, maintainer lines and conventional varieties of *Brassica napus* with a double haploid induction line of rapeseed.
FIG. 2 is a flowchart of breeding of a double haploid induction line of rapeseed.
FIG. 3 is a flowchart of a method for obtaining an early generation stable line of rapeseed.
FIG. 4 is a flowchart of breeding of a double haploid induction line of rapeseed Y3560.
FIG. 5 is a flowchart of breeding of a double haploid induction line of rapeseed Y3380.
FIG. 6 is a flowchart of breeding of an early generation stable line P3-2 of rapeseed.
FIG. 7 is a breeding diagram of a polima CMS restorer line Rong C2859 of *Brassica napus.*
FIG. 8 is a breeding diagram of a GMS restorer line Rong C2994 of *Brassica napus.*
FIG. 9 is a breeding diagram of a polima CMS maintainer line Rong B4653 of *Brassica napus.*
FIG. 10 is a breeding diagram of an ogura CMS restorer line Rong C4707 of *Brassica napus.*
FIG. 11 is a breeding diagram of an ogura CMS maintainer line Rong B Luo 4700 of *Brassica napus.*
FIG. 12 is a flow cytometry ploidy identification diagram of P3-2 tetraploid rapeseed.
FIG. 13 is a flow cytometry ploidy identification diagram of P3-2 tetraploid rapeseed.
FIG. 14 is a flow cytometry ploidy identification diagram of Y3380.
FIG. 15 is a flow cytometry ploidy identification diagram of Y3560.

### DETAILED DESCRIPTION

### Embodiment 1

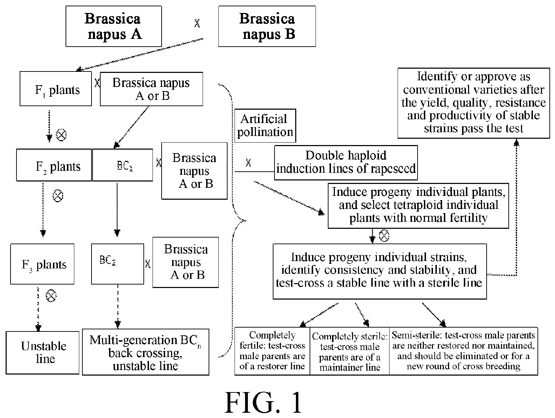

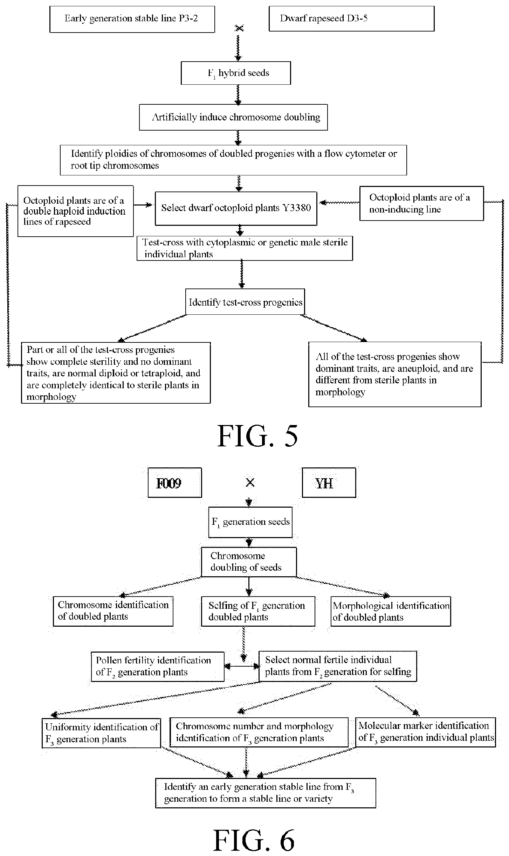

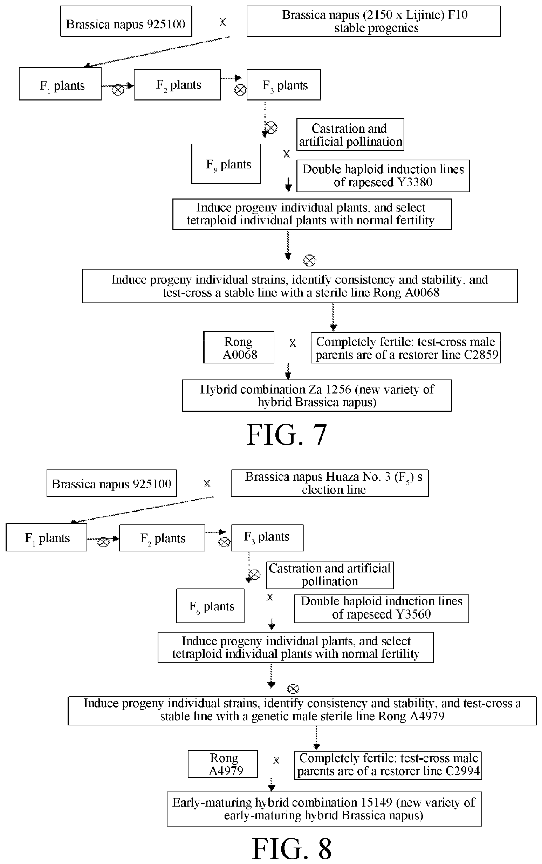

Referring to FIG. 1, FIG. 2, FIG. 5 and FIG. 7, a *Brassica napus* double-low material "925100" was crossed with a restorer material "2150X Lijinte", and excellent individual plants were selfed, separation was still present till the F₉ generation, artificial castration was performed at the F₁₀ generation, pollination was performed with a double haploid induction line of rapeseed Y3380 obtained by the applicant, and a large number of induced progeny seeds were obtained. The F₁₁ generation (induced progenies) was planted and flow cytometry is performed, individual plants with normal fertility and ploidy (tetraploid) and without dominant traits (dwarf) of the inducing line were selfed by bagging, excellent individual strains were test-crossed with a polima cytoplasmic male sterile line "Rong A0068", the test-cross progenies were completely fertile and the traits of the individual plants within the strains were consistent, indicating that the strains were of a restorer line. Therefore, a stable double-low cytoplasmic male sterile restorer line "Rong C2859" of *Brassica napus* was bred after the 2 generation was induced. The "Rong C2859" was crossed with "Rong A0068" to obtain a hybrid combination Za 1256, which passed Sicbuan rapeseed regional test and national regional test in 2014 and 2015, entered the 2016 annual production test, and was a variety to be approved.

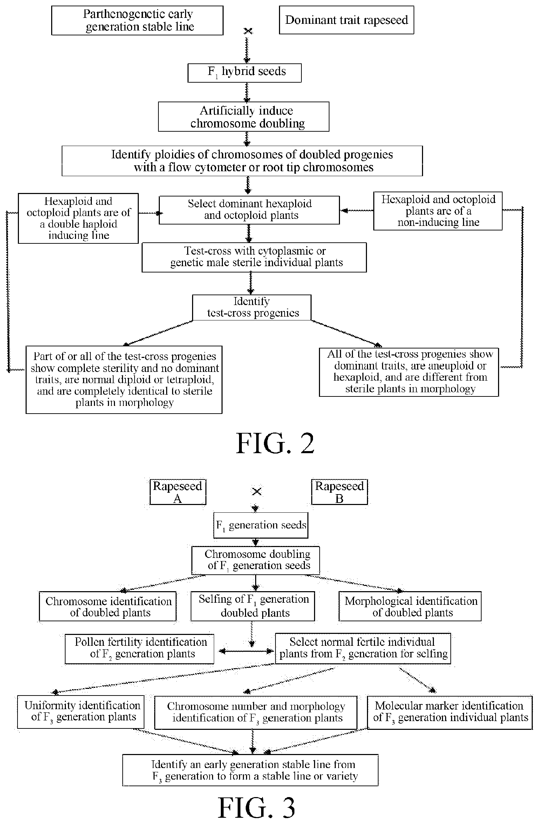

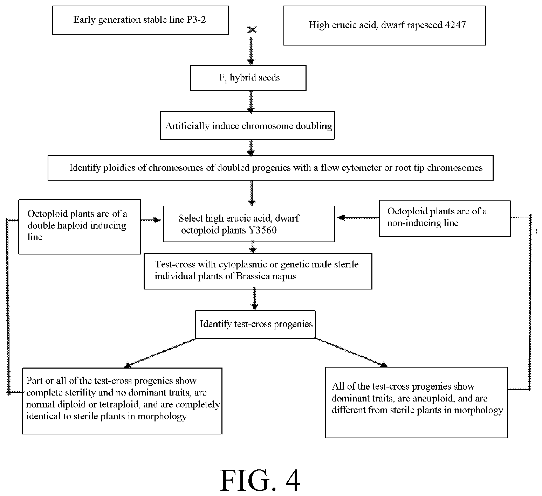

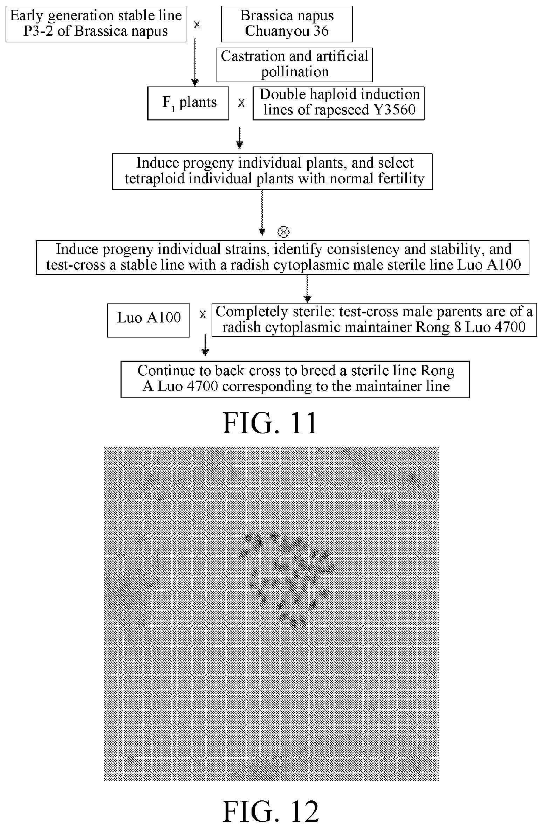

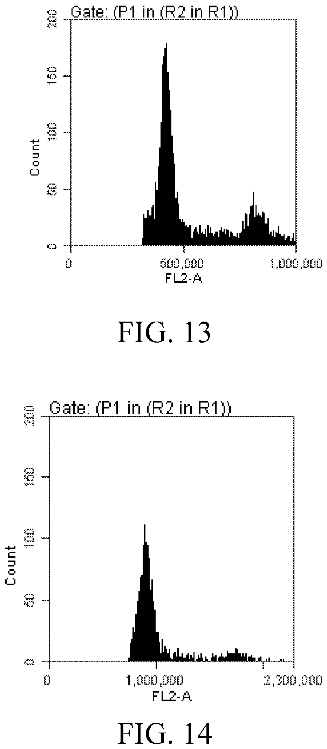

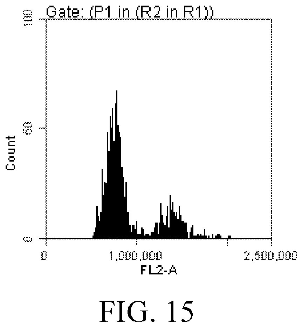

In the present embodiment, the double haploid induction line of rapeseed was obtained by the following method:
Referring to FIG. 2, FIG. 4, FIG. 6, FIG. 12, FIG. 13 and FIG. 15, the tetraploid early generation stable line P3-2 of *Brassica napus* obtained by the applicant was reciprocally crossed with 20 homozygous tetraploid *Brassica napus,* three reciprocal cross F₁ generations were separated, and the combined F₂ generation of the three showed stable strains, indicating that P3-2 has the parthenogenesis genetic characteristic. P3-2 was reciprocally crossed with high erucic acid, dwarf rapeseed 4247 (dwarf and high erucic acid were dominant traits), then hybrid F₁ generation seeds were subjected to chromosome doubling, and doubled progenies were identified by a flow cytometer or root tip microscopic observation to show dwarf octaploid plants named Y3560.

Referring to FIG. 2, FIG. 5, FIG. 6, FIG. 12, FIG. 13 and FIG. 14, the *Brassica napus* tetraploid early generation stable line P3-2 obtained by the applicant was reciprocally crossed with 20 homozygous tetraploid *Brassica napus,* three reciprocal cross F₁ generations were isolated, and the combined F₂ generation of the three showed stable strains, indicating that P3-2 has the parthenogenesis genetic characteristic. P3-2 was reciprocally crossed with tetraploid dwarf *Brassica napus* D3-5 (dwarf was a dominant trait), then hybrid F₁ generation seeds were subjected to chromosome doubling, and doubled progenies were identified by a flow cytometer or root tip microscopic observation to show dwarf octaploid plants named Y3380.

In this embodiment, the specific method of artificial chromosome doubling for hybrid F₁ seeds of P3-2 and dwarf rapeseed D3-5, as well as hybrid F₁ seeds of P3-2 and dwarf, high erucic acid rapeseed 4247 on a medium with colchicine was as follows:
1) disinfecting the surfaces of the seeds with 75% alcohol for 25 seconds, disinfecting same with 0.1% mercury bichloride for 12 minutes, then washing away the mercury bichloride on the surfaces of the seeds with sterile water, sucking the water on the surfaces of the seeds with sterile paper, and then inoculating a first medium (chromosome doubling inducing medium) with the seeds;
2) allowing the seeds to root and sprout on the first medium under the culture conditions: temperature 25°C, daylight illumination 16 hours, light intensity 2000 lux, night dark culture 8 hours, until the plants grew to 1-2 true leaves, and cutting the plants from the hypocotyls for continuing to grow on a second medium;
3) inserting the cut plants into the second medium to continue the culture, and after lateral buds were differentiated, transferring the lateral buds and the plants to a third medium (rooting medium) for rooting culture; and
4) hardening seedlings of the plants at room temperature for 3 days after the plants grew thick roots after two weeks of rooting culture, taking the plants out, washing away the medium on the plants with tap water, soaking the plants in a soaking buffer solution for 15 minutes, and then transplanting the plants to a greenhouse, the greenhouse having a temperature of 25°C and a relative humidity of 60%, which can ensure that the survival rate of transplanting was 95% or above;
the first medium consisted of the following components:

| | |
|---|---|
| MS medium | 1 L |
| 6-benzyl adenine (6BA) | 0.5 mg |
| colchicine | 50 mg |
| sucrose | 20 g |
| agar | 8 g, |

the pH value of the first medium was 5.8-6.0;
the MS medium was invented by Murashige and Skoog, abbreviated as MS, and its formulation was shown in annexed Table 1.
the second medium consisted of the following components:

| | |
|---|---|
| MS medium | 1 L |
| 6-benzyl adenine (6BA) | 0.5 mg |
| colchicine | 30 mg |
| sucrose | 30 g |
| agar | 8 g, |

the pH value of the second medium was 5.8-6.0;
the third medium consisted of the following components:

| | |
|---|---|
| MS medium | 1 L |
| α-naphthaleneacetic acid | 0.03 mg |
| colchicine | 20 mg |
| sucrose | 20 g |
| agar | 8 g, |

the pH value of the third medium was 5.8-6.0;
the soaking buffer solution consisted of the following components:

| | |
|---|---|
| water | 1 L |
| famoxadone or curzate | 0.6 g |
| α-naphthaleneacetic acid | 0.5 mg. |

Referring to FIG. 2, FIG. 3 and FIG. 5, Y3380 as male parents was test-crossed with a cytoplasmic male sterile line (0464A) of *Brassica napus* to obtain 50 test-cross progenies, all of which had high stalks and were tetraploid *Brassica napus,* where 49 strains were completely sterile, 1 strain was semi-sterile, and the morphological characteristics were completely identical to the 0464A. At the same time, hybrid F₁ (non-doubled strains) of P3-2 and dwarf rapeseed D3-5 was used as male parents and test-crossed with 0464A as a contrast for verification to obtain 102 test-cross progenies, which included 62 dwarf plants and 40 high-stalk plants, and were high in fertility separation with 73 completely fertile, 20 semi-sterile and 9 completely sterile. It indicated that the genes in Y3380 did not enter the test-cross plants, the test-cross progenies were produced by parthenogenesis of the 0464A, and the induction rate was 98%. Y3380 as male parents was convergently crossed with castrated *Brassica napus* 3954 (3954 was F₁, obtained by crossing Zhongshuang 11 with CAX), the convergently crossed progenies F₁ were separated, each F₁ was selfed, and 45 F₁ selfed strains were harvested. 45 F₂ generation strains were planted, and 45 stable strains appeared, so that the stable strains showed 100% and the induction rate was 100%.

Y3380 as male parents was convergently crossed with castrated *Brassica napus* 3968 (3968 was F₁, obtained by crossing Zhongshuang 11 with 1365), the convergently crossed progenies F₁ were separated, each F₁ was selfed, and 52 F₁ selfed strains were harvested. 52 F₂ generation strains were planted, and 28 stable strains appeared, so that the stable strains showed 53.85% and the induction rate was 53.85%.

Y3380 as male parents was crossed with castrated *Brassica napus* Zhongshuang 11 (conventional variety, homozygous line) to obtain 70 hybrid F₁ plants, the 70 F₁ plants were completely identical to Zhongshuang 11 in morphology, and the F₂ generation did not separate after each individual plant was selfed, and showed stable strains that were completely identical to Zhongshuang 11 in morphology, indicating that the F₁ generation was homozygous. That is, the crossing process of Y3380 and Zhongshuang 11 induced parthenogenesis in Zhongshuang 11, and the F₁ produced was of parthenogenetic selfing and was homozygous, so that F₁ was stable, F₂ was also stable, F₁ and F₂ were completely identical to Zhongshuang 11 in morphology, and the induction rate was 100%.

Similarly, Y3380 as male parents was crossed with castrated *Brassica campestris* Ya'an yellow rapeseed YH (diploid rapeseed, 2n=20) to obtain 98 hybrid F₁ plants, in which 97 F1 plants were completely identical to YH in morphology, and the F₂ generation after each individual plant was selfed was diploid and identical to YH in morphology, indicating that the crossing process of Y3380 and YH induced parthenogenesis in YH, the F₁ produced was of parthenogenetic selfing and completely identical to YH in morphology, and the induction rate was 98.9%. Finally, dominant dwarf octaploid plants Y3380 were identified as a double haploid induction line of rapeseed.

Referring to FIG. 2, FIG. 3 and FIG. 4, Y3560 as male parents was test-crossed with a cytoplasmic male sterile line (0464A) of *Brassica napus* to obtain 80 test-cross progenies, all of which had high stalks, 76 plants were tetraploid *Brassica napus,* 2 plants were diploid and 2 plants were octaploid, where the 76 tetraploid plants were completely sterile, the 4 plants were semi-sterile, and the morphological characteristics were completely identical to the 0464A. At the same time, hybrid F₁ (non-doubled strains) of P3-2 and dwarf, high erucic acid rapeseed 4247 was used as male parents and test-crossed with 0464A as a contrast for verification to obtain 153 test-cross progenies, which included 102 dwarf plants and 51 high-stalk plants, and were high in fertility separation with 65 completely fertile, 35 semi-sterile and 53 completely sterile. It indicated that the genes in Y3560 did not enter the test-cross plants, the test-cross progenies were produced by parthenogenesis of the 0464A, and the induction rate was 95%.

Y3560 as male parents was crossed with castrated *Brassica campestris* Ya'an yellow rapeseed YH (diploid rapeseed, 2n=20) to obtain 145 hybrid F₁ plants, in which 143 F₁ plants were completely identical to YH in morphology, and the F₂ generation after each individual plant was selfed was diploid and identical to YH in morphology, indicating that the crossing process of Y3560 and YH induced parthenogenesis in YH, the F₁ produced was of parthenogenetic selfing and completely identical to YH in morphology, and the induction rate was 98.6%.

Similarly, Y3560 as male parents was crossed with castrated *Brassica juncea* GW (tetraploid rapeseed, 2n=36) to obtain 124 hybrid F₁ plants, in which 123 F₁ plants were completely identical to GW in morphology, and the F₂ generation after each individual plant was selfed was tetraploid and identical to GW in morphology, indicating that the crossing process of Y3560 and GW induced parthenogenesis in GW, the F₁ produced was of parthenogenetic selfing and completely identical to GW in morphology, and the induction rate was 99.2%. Finally, dominant dwarf octaploid plants Y3560 were identified as a double haploid induction line of rapeseed.

Referring to FIG. 3, FIG. 6, FIG. 12 and FIG. 13, the method for obtaining the early generation stable line P3-2 was as follows:
performing artificial castrated crossing on *Brassica napus* F009 (tetraploid, chromosomes 2n=38) and *Brassica campestris* YH (diploid, Ya'an yellow rapeseed, chromosomes 2n=20) from which buds were peeled to obtain F₁ generation hybrid seeds; performing artificial chromosome doubling on the F₁ generation hybrid seeds with colchicine on a medium; selfing (or forcedly selfing) doubled F₁ generation plants to obtain an F₂ generation, performing field planting observation on the F₂ generation, and identifying the fertility by dyeing pollen with acetic acid magenta to judge the fertility of the pollen, where three cases may occur (1. haploid plants, with little pollen and extremely low fertility; 2. polyploid plants completely sterile, with the development of floral organs impaired, failing to flower normally, having no pollen; 3. normal fertile plants, with more pollen, pollen fertility 95% or more); selfing normal fertile plants of the F₂ generation to obtain an F₃ generation; identifying the homozygosity of the F₃ generation, and planting individual plants of the F₃ generation, where 32% of the fertile individual plants were uniform and normal in flowering and seed setting; performing cytological identification on the uniform plants, showing that the number of chromosomes was consistent (38) and the chromosome morphology was normal; marking with SSR molecular markers, performing DNA polymerase chain reaction, observing the DNA band type of each individual plant by electrophoresis under the amplification of each specific primer, showing that each individual plant was a hybrid progeny of F009 and YH, and the number and type of DNA amplification bands of the individual plants were consistent, and it can be judged that these plants were homozygous, that is, early generation stable lines; and naming one of the early generation stable lines of *Brassica napus* (38 chromosomes) with large leaves, no cleft leaves, compact leave and an oil content of 55% as P3-2.

In the present embodiment, the specific method of performing artificial chromosome doubling on the F₁ generation hybrid seeds with colchicine on a medium was as follows:
1) disinfecting the surfaces of the seeds with 75% alcohol for 25 seconds, disinfecting same with 0.1% mercury bichloride for 12 minutes, then washing away the mercury bichloride on the surfaces of the seeds with sterile water, sucking the water on the surfaces of the seeds with sterile paper, and then inoculating a first medium (chromosome doubling inducing medium) with the seeds;
2) allowing the seeds to root and sprout on the first medium under the culture conditions: temperature 25°C, daylight illumination 16 hours, light intensity 2000 lux, night dark culture 8 hours, until the plants grew to 1-2 true leaves, and cutting the plants from the hypocotyls for continuing to grow on a second medium;
3) inserting the cut plants into the second medium to continue the culture, and after lateral buds were differentiated, transferring the lateral buds and the plants to a third medium (rooting medium) for rooting culture; and
4) hardening seedlings of the plants at room temperature for 3 days after the plants grew thick roots at two weeks of rooting culture, taking the plants out, washing away the medium on the plants with tap water, soaking the plants in a soaking buffer solution for 15 minutes, and then transplanting the plants to a greenhouse, the greenhouse having a temperature of 25°C and a relative humidity of 60%, which can ensure that the survival rate of transplanting was 95% or above;
the first medium consisted of the following components:

| | |
|---|---|
| MS medium | 1 L |
| 6-benzyl adenine (6BA) | 0.5 mg |
| colchicine | 30 mg |
| sucrose | 20 g |
| agar | 8 g, |

the pH value of the first medium was 5.8-6.0;
the MS medium was invented by Murashige and Skoog, abbreviated as MS, and its formulation was shown in annexed Table 1.
the second medium consisted of the following components:

| | |
|---|---|
| MS medium | 1 L |
| 6-benzyl adenine (6BA) | 0.5 mg |
| colchicine | 20 mg |
| sucrose | 30 g |
| agar | 8 g, |

the pH value of the second medium was 5.8-6.0;
the third medium consisted of the following components:

| | |
|---|---|
| MS medium | 1 L |
| α-naphthaleneacetic acid | 0.03 mg |
| colchicine | 5 mg |
| sucrose | 20 g |
| agar | 8 g, |

the pH value of the third medium was 5.8-6.0;
the soaking buffer solution consisted of the following components:

| | |
|---|---|
| water | 1 L |
| famoxadone or curzate | 0.6 g |
| α-naphthaleneacetic acid | 0.5 mg. |

**Table 1 MS medium ingredients**

| Ingredient | Molecular weight | Concentration (mg/L) |
|---|---|---|
| Major element | | |
| Potassium nitrate KNO3 | 101.21 | 1900 |
| Ammonium nitrate NH4NO3 | 80.04 | 1650 |
| Potassium dihydrogen phosphate KH2PO4 | 136.09 | 170 |
| Magnesium sulfate MgSO4•7H2O | 246.47 | 370 |
| Calcium chloride CaCl2•2H2O | 147.02 | 440 |

| Trace element | | |
|---|---|---|
| Potassium iodide KI | 166.01 | 0.83 |
| Boric acid H3BO3 | 61.83 | 6.2 |
| Manganese sulfate MnSO4•4H2O | 223.01 | 22.3 |
| Zinc sulfate ZnSO4•7H2O | 287.54 | 8.6 |
| Sodium molybdate Na2MoO4•2H2O | 241.95 | 0.25 |
| Copper sulfate CuSO4•5H2O | 249.68 | 0.25 |
| Cobalt chloride CoCl2•6H2O | 237.93 | 0.025 |

| Iron salt | | |
|---|---|---|
| Disodium edetate Na2.EDTA | 372.25 | 37.25 |
| Ferrous sulfate FeSO24•7H2O | 278.03 | 27.85 |

| Organic ingredients | | |
|---|---|---|
| Inositol | | 100 |
| Glycine | | 2 |
| Thiamine hydrochloride VB 1 | | 0.1 |
| Pyridoxine hydrochloride VB6 | | 0.5 |
| Niacin VB5 or VPP | | 0.5 |
| Sucrose | 342.31 | 30 g/L |
| pH | 5.8-6.0 | |

### Embodiment 2:

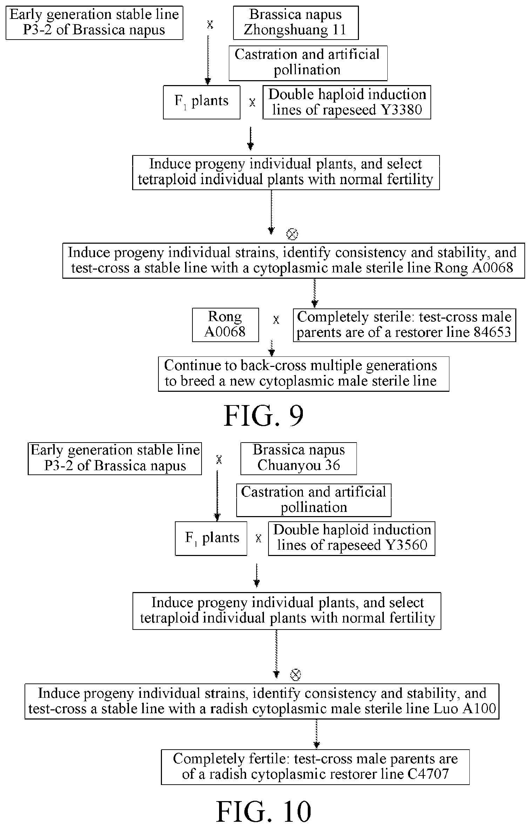

Referring to FIG. 1, FIG. 2, FIG. 5 and FIG. 9, the *Brassica napus* early generation stable line P3-2 was crossed with Zhongshuang 11, the hybrid progenies F₁ were artificially castrated and pollinated with the double haploid induction line of rapeseed Y3380 obtained by the applicant, the induced progenies were selfed by bagging, the F₂ generation (induced progenies) was planted and subjected to flow cytometry, individual plants with normal fertility and ploidy (tetraploid) and without dominant traits (dwarf) of the inducing line were selfed by bagging, the purity of the F₃ generation was identified, stable strains 4653 were selected and test-crossed with the polima CMS line "Rong A0068", and the test-cross progenies were completely sterile, indicating that the induced stable strains 4653 were of a maintainer line, where the polima CMS maintainer line had high oil content (49% or more), good lodging and disease resistance, early maturity and complete infertility, and the sterile progenies were less affected by temperature, and are currently undergoing multi-generation back crossing to replace nuclear genes of the sterile line to form a sterile line Rong A4653 corresponding to the Rong B4653 maintainer line.

### Embodiment 3:

Referring to FIG. 1, FIG. 2, FIG. 4 and FIG. 10, *Brassica napus* Chuanyou 36 that was not easily obtained from restorer genes of ogura CMS was radish cytoplasmic three-line hybrid rapeseed approved in the upper, middle and lower reaches of the Yangtze River in Sichuan Province, which contained radish cytoplasmic restorer genes. Chuanyou 36 was crossed with P3-2 to obtain a convergent cross F₁, and the F₁ was castrated and pollinated with the double haploid induction line of rapeseed Y3560 obtained by the applicant. The F₂ generation (induced progenies) was planted and subjected to flow cytometry, individual plants with normal fertility and ploidy (tetraploid) and without dominant traits (dwarf) of the inducing line were selfed by bagging, the purity of the F₃ generation was identified, stable strains 4707 were obtained, at the same time, the pollen of 4707 was test-crossed with the stable radish cytoplasmic male sterile line Luo A100, the test-cross progenies were completely fertile, indicating that the test-cross male parent 4707 was a restorer line of the sterile line, and a *Brassica napus* ogura CMS restorer line Rong C4707 was formed. The restorer line restores the radish cytoplasmic male sterile line thoroughly, and has double low quality, lodging resistance, disease resistance, and oil content of 45% or more.

### Embodiment 4:

Referring to FIG. 1, FIG. 2, FIG. 4 and FIG. 11, common rapeseed was a maintainer line of the radish cytoplasmic male sterile line, Chuanyou 36 was crossed with P3-2 to obtain a convergent cross F₁, and the F₁ was pollinated with the double haploid induction line of rapeseed Y3560 obtained by the applicant. The F₂ generation (induced progenies) was planted and subjected to flow cytometry, individual plants with normal fertility and ploidy (tetraploid) and without dominant traits (dwarf) of the inducing line were selfed by bagging, the purity of the F₃ generation was identified, stable strains 4700 were obtained, at the same time, the pollen of 4700 was test-crossed with the stable radish cytoplasmic male sterile line Luo A100, the test-cross progenies were completely sterile, indicating that the test-cross male parent 4700 was a maintainer line of the sterile line, where the maintainer line was stable in selfing, did not separate sterile plants, had a high oil content (47%), double low quality, lodging resistance and disease resistance, and was being back-crossed with the Luo A100 sterile line to replace nuclear genes of the sterile line to form a sterile line Rong A Luo 4700 consistent with Rong B Luo 4700 in nuclear genes.

### Embodiment 5:

Referring to FIG. 1, FIG. 2, FIG. 4 and FIG. 8, a *Brassica napu* double-low material "925100" was crossed with "Huaza No. 3 selection line (F₅)", and excellent individual plants were selfed. The F₂ generation was still separable, the F₆ generation was artificially castrated and pollinated with the double haploid induction line of rapeseed Y3560 obtained by the applicant, the F₇ generation (induced progenies) was planted and subjected to flow cytometry, individual plants with normal fertility and ploidy (tetraploid) and without dominant traits (dwarf) of the inducing line were selfed by bagging, excellent individual plants were selected and test-crossed with a genetic male sterile (GMS) line "Rong A4979", the test-cross progenies were completely fertile and the individual strains were consistent in traits, indicating that the strains were of a GMS restorer line. Therefore, a stable GMS restorer line "Rong C2994" of double-low *Brassica napus* was bred after the 2 generation was induced. The restorer line was combined with the "Rong A4979" to produce a hybrid combination Za 15149 of *Brassica napus,* which was an early maturity combination and is currently undergoing the first-year regional trial.

The breeding method of the double haploid induction line of rapeseed in the above embodiments was the same as that in Embodiment 1.

The above embodiments further illustrate the above description of the present invention, but it should not be understood that the scope of the present invention is limited to the above embodiments. The techniques implemented based on the above all fall within the scope of the present invention.

## Claims

1. A method for breeding *Brassica napus* varieties and materials with a double haploid induction line of rapeseed, comprising the following steps:
1) determining target traits of breeding restorer lines, maintainer lines and conventional varieties of *Brassica napus,* crossing or convergently crossing at least two *Brassica napus* with the target traits, and performing back crossing or multi-generation back crossing according to the requirements of the target traits to form cross progenies, convergent cross progenies or back-cross progenies;
2) artificially castrating buds of the cross, convergent cross or back-cross progeny materials obtained in step 1) at the flowering stage, and performing bagging isolation;
3) artificially pollinating the plants within 2 to 4 days after castration in step 2) with pollen of the double haploid induction line of rapeseed, performing bagging isolation, and harvesting pollinated induced seeds;
4) planting induced seeds obtained in step 3), identifying the ploidies with a flow cytometer at the seedling stage to eliminate polyploids, haploids or plants with dominant characters of the double haploid induction line of rapeseed, selecting tetraploid plants with normal fertility, and bagging and selfing individual plants;
5) performing strain planting on individual selfing seeds in step 4), investigating the morphologic consistency of the strains, and identifying the consistency and stability of the strains through molecular markers;
6) test-crossing the stable tetraploid strains identified in step 5) with a *Brassica napus* cytoplasmic male sterile line, or with a *Brassica napus* genetic male sterile line, identifying the fertility of the test-cross progenies, and judging the restoring and maintaining relationship of the test-cross male parents;
7) determining that the corresponding test-cross male parents are of a maintainer line if the test-cross progenies in step 6) are completely sterile, and are of a restorer line if the test-cross progenies are completely fertile;
8) continuing to back-cross the maintainer line identified in step 7) with a sterile line by multiple generations to breed a stable sterile line consistent with the maintainer line in nuclear genes; directly test-matching the restorer line identified in step 7) with a sterile line of a corresponding system to breed a hybrid combination, and performing variety comparison test on the hybrid combination, wherein the variety that has yield, resistance, productivity and quality traits better than other large-area varieties in production and meets the variety identification or approval standards can form a hybrid rapeseed variety, which can be promoted and applied in production by identification or approval of provincial or national seed management departments; and
9) performing comparison and production trials on the stable tetraploid strains obtained in step 6), wherein the variety that has yield, resistance, productivity and quality traits superior to the varieties applied in large scale during production and meets the variety identification or approval standards can form a conventional variety, which can be promoted and applied in production by identification or approval of provincial or national seed management departments;
a method for breeding the above-mentioned double haploid induction line of rapeseed, comprising the following steps:
(1) breeding an early generation stable line with the parthenogenesis genetic characteristic:
a. artificially doubling chromosomes of hybrid F₁ generation seeds of two rapeseed parent materials on a medium by using a chromosome doubling inducer to obtain doubled F₁ generation plants;
b. selfing or forcedly selfing the doubled F₁ generation plants to obtain an F₂ generation, performing field planting observation on the F₂ generation, identifying the fertility of each individual plant, selecting fertile progenies and selfing same to obtain an F₃ generation, identifying the homozygosity of the F₃ generation by morphology, cytology and molecular markers, performing polymerase chain reaction amplification on progenies DNA, and observing the type and number of DNA bands of the individual plants under the amplification of each specific primer by electrophoresis, which shows that each individual plant is a hybrid progeny of two parents, and the molecular marker maps of the individual plants are consistent, indicating that these individual plants are of a homozygous line, i.e. an early generation stable line;
c. reciprocally crossing the obtained early generation stable line with at least 10 conventional homozygous stable lines of rapeseed, and identifying the genetic characteristics of the early generation stable line at the F₁ and F₂ generations, i.e., identifying whether there is the parthenogenesis characteristic, wherein if F₁ is separated and part of stable strains appear in the F₂ generation in the reciprocal crossing, the corresponding early generation stable line is an early generation stable line with the parthenogenesis genetic characteristic;
(2) breeding polyploid rapeseed with dominant genetic traits, parthenogenesis genetic characteristic and ploidy genetic stability:
a. crossing the early generation stable line with the parthenogenesis genetic characteristic with rapeseed with dominant traits to obtain hybrid F₁ generation seeds, and artificially doubling chromosomes of the hybrid F₁ seeds on a medium by using a chromosome doubling inducer to obtain doubled F₁ plants with dominant traits;
b. identifying the chromosome ploidies of the doubled F₁ plants with dominant traits through microscopic observation or a flow cytometer, selecting polyploid plants with dominant traits, and eliminating abnormal doubled plants, aneuploid plants and doubled plants without dominant traits, the polyploid plants with dominant traits being mainly hexaploid or octoploid rapeseed plants with ploidy genetic stability, good setting property, parthenogenesis genetic characteristic and dominant traits;
(3) identifying the double haploid induction line of rapeseed and measuring the inducing capability:
a. the dominant traits in the polyploid plants with ploidy genetic stability, parthenogenesis genetic characteristic and dominant traits can be used for removing hybrid plants generated in the test-cross progenies, and if dominant plants or aneuploid plants appear in the test-cross progenies, it indicates that the plants are generated by the polyploid plants and female parents and are removed; and
b. if the individual test-cross progenies are completely sterile but have normal ploidies, i.e. diploid or tetraploid rapeseed, and do not have dominant traits, it indicates that the genes of the corresponding male parents of the test-cross progenies do not enter the test-cross progenies, wherein the dominant polyploid plants are of the double haploid induction line of rapeseed.

2. The method for breeding *Brassica napus* varieties and materials with a double haploid induction line of rapeseed according to claim 1, wherein the double haploid induction line of rapeseed is bred by artificially doubling chromosomes of hybrid F₁ generation seeds of two parent materials, or hybrid F₁ generation seeds obtained by crossing the early generation stable line with the parthenogenesis genetic characteristic with rapeseed with dominant traits, on a medium by using a chromosome doubling inducer, and the specific method is as follows:
1) disinfecting the surfaces of the seeds with 75% alcohol for 25-40 seconds, disinfecting same with 0.1% mercury bichloride for 12-17 minutes, then washing away the mercury bichloride on the surfaces of the seeds with sterile water, sucking the water on the surfaces of the seeds with sterile paper, and then inoculating a first medium with the seeds;
2) allowing the seeds to root and sprout on the first medium under the culture conditions: temperature 23-25°C, daylight illumination 12-16 hours, light intensity 2000-3000 lux, night dark culture 8-12 hours, until the plants grow to 1-2 true leaves, and cutting the plants from the hypocotyls for continuing to grow on a second medium;
3) inserting the cut plants into the second medium to continue the culture, and after lateral buds are differentiated, transferring the lateral buds and the plants to a third medium for rooting culture; and
4) hardening seedlings of the plants at room temperature for 3-7 days after the plants grow thick roots after two weeks of rooting culture, taking the plants out, washing away the medium on the plants with tap water, soaking the plants in a soaking buffer solution for 15-30 minutes, and then transplanting the plants to a greenhouse, the greenhouse having a temperature of 16-25°C and a relative humidity of 60-80%, which can ensure that the survival rate of transplanting is 95% or above;
the first medium consists of the following components:
| | |
|---|---|
| MS medium | 1 L |
| 6-benzyl adenine | 0.5-1.5 mg |
| chromosome doubling inducer | 30-70 mg |
| sucrose | 20-30 g |
| agar | 8-10 g, |
the pH value of the first medium is 5.8-6.0;
the second medium consists of the following components:
| | |
|---|---|
| MS medium | 1 L |
| 6-benzyl adenine | 0.5-1 mg |
| chromosome doubling inducer | 20-40 mg |
| sucrose | 20-30 g |
| agar | 8-10 g, |
the pH value of the second medium is 5.8-6.0;
the third medium consists of the following components:
| | |
|---|---|
| MS medium | 1 L |
| α-naphthaleneacetic acid | 0.03-0.5 mg |
| chromosome doubling inducer | 5-20 mg |
| sucrose | 20-30 g |
| agar | 8-10 g, |
the pH value of the third medium is 5.8-6.0;
the soaking buffer solution consists of the following components:
| | |
|---|---|
| water | 1L |
| famoxadone or curzate | 0.6-1.2 g |
| α-naphthaleneacetic acid | 0.5-1 mg. |

3. The method for breeding *Brassica napus* varieties and materials with a double haploid induction line of rapeseed according to claim 1 or 2, wherein the chromosome doubling inducer is at least one of colchicine, trifluralin and oryzalin.
